# EUROPEAN PATENT APPLICATION

(11) **EP 4 726 030 A1**
(43) Date of publication of application: **15.04.2026**
(21) Application number: 24825978.0
(22) Date of filing: 20.06.2024
(51) Int. Cl.: C12N 5/0786, C12M 1/00, C12M 3/00, C12N 5/0789

(54) **METHOD FOR PRODUCING HUMAN MACROPHAGE, DIFFERENTIATION-INDUCING AGENT, DIFFERENTIATION-INDUCING KIT, METHOD FOR INDUCING DIFFERENTIATION OF HUMAN MACROPHAGE, PROLIFERATION-PROMOTING AGENT FOR HUMAN MACROPHAGE, KIT FOR PROMOTING PROLIFERATION OF HUMAN MACROPHAGE, METHOD FOR PROLIFERATING HUMAN MACROPHAGE, AND HUMAN MACROPHAGE**

(30) Priority: 21.06.2023 JP 2023101497
(71) Applicant: Kagoshima University, Kagoshima-shi, Kagoshima 890-8580 (JP)
(72) Inventor: HARA, Hiromitsu, Kagoshima-shi, Kagoshima 890-8580 (JP); MATSUMOTO, Shin-ei, Kagoshima-shi, Kagoshima 890-8580 (JP)
(74) Representative: v. Bezold & Partner Patentanwälte - PartG mbB
(86) International application number: PCT/JP2024/022414
(87) International publication number: WO 2024/262577

(57) **Abstract**

A method of producing human macrophages includes culturing human hematopoietic progenitor cells in a medium that substantially does not contain either a macrophage colony-stimulating factor or a granulocyte-macrophage colony-stimulating factor, in presence of a TREM2 signal activator.

## Description

### Technical Field

The present disclosure relates to a method of producing human macrophages, a differentiation-inducing agent, a differentiation-inducing kit, a method of inducing differentiation into human macrophages, a proliferation-promoting agent for promoting proliferation of human macrophages, a proliferation-promoting kit for promoting proliferation of human macrophages, a method of proliferating human macrophages, and human macrophages.

### Background Art

When mature blood cells are induced from hematopoietic progenitor cells of bone marrow in vitro, it is necessary to add colony-stimulating factors, growth factors, hematopoietic factors, cytokines such as lymphokines, sustentacular cells such as bone marrow stromal cells, cell matrices, or the like. Macrophages, that is, leukocytes that contribute to protection against infections and maintenance of tissue homeostasis are derived from hematopoietic stem cells in the bone marrow, and their differentiation requires stimulation by a macrophage colony-stimulating factor (M-CSF) or a granulocyte-macrophage colony-stimulating factor (GM-CSF). A method has been established for inducing differentiation of cells having characteristics of monocytes and macrophages (bone marrow derived macrophages: BMDMs) by culturing human bone marrow cells or peripheral blood leukocytes in the presence of M-CSF or GM-CSF. It is also known that interleukin-34 (IL-34) acts on an M-CSF receptor to promote differentiation from monocytes into macrophages and proliferation of macrophages (refer to Non Patent Literatures 1 and 2).

Furthermore, a known method involves culturing induced pluripotent stem cells (iPSCs) on culture plates coated with extracellular matrices, continuously culturing them in a medium supplemented with multiple cytokines and growth factors such as BMP4, VEGF, stem cell factor (SCF), bFGF, Flt3 ligand, interleukin-3 (IL-3), thrombopoietin (TPO), M-CSF, and GM-CSF, to induce differentiation into monocytes, and further culturing the induced monocytes with M-CSF to differentiation-induce into mature macrophages (see Non Patent Literature 3).

As a differentiation-inducing agent that induces differentiation into macrophages from monocytes without use of M-CSF, Patent Literature 1 discloses an differentiation-inducing agent, an agent containing a compound having antagonistic action on angiotensin II receptor, and activating action on peroxisomal proliferator-activated receptor γ.

### Citation List

### Patent Literature

Patent Literature 1: Unexamined Japanese Patent Application Publication No. 2015-47125

### Non Patent Literature

Non Patent Literature 1: Suwen Wei and eight others, "Functional overlap but differential expression of CSF-1 and IL-34 in their CSF-1 receptor-mediated regulation of myeloid cells", Journal of Leukocyte Biology, 2010, 88, 495-505
Non Patent Literature 2: Haishan Lin and 16 others, "Discovery of a Cytokine and Its Receptor by Functional Screening of the Extracellular Proteome", SCIENCE, 2008, 320, 807-811
Non Patent Literature 3: Masakatsu D. Yanagimachi and 14 others, "Robust and Highly-Efficient Differentiation of Functional Monocytic Cells from Human Pluripotent Stem Cells under Serum- and Feeder Cell-Free Conditions", PLOS ONE, 2013, 8(4), e59243

### Summary of Invention

### Technical Problem

In the method of differentiation induction into mature macrophages from hematopoietic progenitor cells using M-CSF, GM-CSF, or IL-34 described above, it is essential to add one of these cytokines to the medium. The bone marrow derived macrophages (BMDMs) obtained by this method do not have proliferative capacity and therefore cannot be expanded in culture. Patent Literature 1 does not examine the survival period of the macrophages obtained by the differentiation-inducing agent, the proliferative characteristics after differentiation or the like. Furthermore, in a case where mature macrophages are differentiation-induced from iPSCs as disclosed in Non Patent Literature 3, it is necessary to use extracellular matrices, multiple types of cytokines and growth factors, and to further culture the induced monocytes with M-CSF.

In view of the above circumstances, an objective of the present disclosure is to provide a method of producing human macrophages, a differentiation-inducing agent, a differentiation-inducing kit, a method of inducing differentiation into human macrophages, a proliferation-promoting agent for promoting proliferation of human macrophages, a proliferation-promoting kit for promoting proliferation of human macrophages, and a method of proliferating human macrophages, capable of acquiring human macrophages that can be induced from hematopoietic progenitor cells in a medium without adding cytokines to the medium, have proliferative capacity after induction, and can be expanded in culture. An another objective of the present disclosure is to provide human macrophages that can be induced from hematopoietic progenitor cells in a medium without adding cytokines to the medium, have proliferative capacity after induction, and can be expanded in culture.

### Solution to Problem

Findings that macrophage-like cells appear when human hematopoietic progenitor cells are stimulated with a ligand of triggering receptor expressed on myeloid cells 2 (TREM2) or a lipid recognition receptor have enabled the present inventors to complete the present disclosure.

### Method of Producing Human Macrophages

A method of producing human macrophages according to the present disclosure includes culturing human hematopoietic progenitor cells in a medium that substantially does not contain either a macrophage colony-stimulating factor or a granulocyte-macrophage colony-stimulating factor, in presence of a TREM2 signal activator.

It is also acceptable that the medium substantially does not contain interleukin-34.

It is also acceptable that the medium substantially does not contain cytokine.

It is also acceptable that, in the culturing, the human hematopoietic progenitor cells are cultured under a condition in which neither a sustentacular cell nor an extracellular matrix is added.

It is also acceptable that the TREM2 signal activator is a lipid.

It is also acceptable that the TREM2 signal activator is a lipid derived from brain or a lipid having a plurality of long-chain fatty acid groups.

It is also acceptable that the TREM2 signal activator is a compound represented by Formula (I), an ester thereof, or a salt thereof (wherein the compound has 60 to 90 carbon atoms, R¹ represents a saturated or unsaturated aliphatic hydrocarbon group, and R² represents a saturated or unsaturated aliphatic hydrocarbon group optionally containing a ring structure or a substituent).

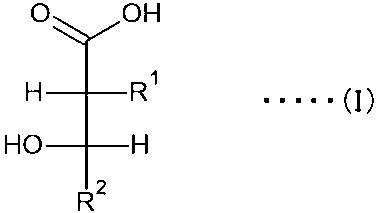

It is also acceptable that the TREM2 signal activator is a phospholipid.

It is also acceptable that the phospholipid is a lipid that constitutes a biological membrane.

It is also acceptable that the phospholipid is cardiolipin.

It is also acceptable that a surface of a cell culture vessel for culturing the human hematopoietic progenitor cells is coated with the TREM2 signal activator.

It is also acceptable that the human hematopoietic progenitor cells are human myeloid progenitor cells.

### Differentiation-Inducing Agent

A differentiation-inducing agent that induces differentiation from human hematopoietic progenitor cells into human macrophages according to the present disclosure contains a TREM2 signal activator, wherein the differentiation-inducing agent substantially does not contain either a macrophage colony-stimulating factor or a granulocyte-macrophage colony-stimulating factor.

### Differentiation-Inducing Kit

A differentiation-inducing kit that induces differentiation from human hematopoietic progenitor cells into human macrophages according to the present disclosure includes a cell culture vessel having a surface coated with a TREM2 signal activator, wherein the differentiation-inducing kit is used for culturing the human hematopoietic progenitor cells in a medium that substantially does not contain either a macrophage colony-stimulating factor or a granulocyte-macrophage colony-stimulating factor.

### Method of Inducing Differentiation into Human Macrophages

A method of inducing differentiation into human macrophages according to the present disclosure includes culturing human hematopoietic progenitor cells in a medium that substantially does not contain either a macrophage colony-stimulating factor or a granulocyte-macrophage colony-stimulating factor, in presence of a TREM2 signal activator.

### Proliferation-Promoting Agent for Promoting Proliferation of Human Macrophages

A proliferation-promoting agent for promoting proliferation of human macrophages according to the present disclosure contains a TREM2 signal activator, wherein the proliferation-promoting agent substantially does not contain either a macrophage colony-stimulating factor or a granulocyte-macrophage colony-stimulating factor.

### Proliferation-promoting Kit for Promoting Proliferation of Human Macrophages

A proliferation-promoting kit that promotes proliferation of human macrophages according to the present disclosure includes a cell culture vessel having a surface coated with a TREM2 signal activator, wherein the proliferation-promoting kit is used for culturing human hematopoietic progenitor cells in a medium that substantially does not contain either a macrophage colony-stimulating factor or a granulocyte-macrophage colony-stimulating factor.

### Method of Proliferating Human Macrophages

A method of proliferating human macrophages according to the present disclosure includes culturing human hematopoietic progenitor cells in a medium that substantially does not contain either a macrophage colony-stimulating factor or a granulocyte-macrophage colony-stimulating factor, in presence of a TREM2 signal activator.

### Human Macrophages

A human macrophage according to the present disclosure is capable of proliferating after 10 days or more of successive culture in a medium that substantially does not contain either a macrophage colony-stimulating factor or a granulocyte-macrophage colony-stimulating factor, in presence of a TREM2 signal activator.

A human macrophage according to the present disclosure is a human macrophage of which differentiation is induced dependently on TREM2 by culturing human hematopoietic progenitor cells in a medium that substantially does not contain either a macrophage colony-stimulating factor or a granulocyte-macrophage colony-stimulating factor, in presence of a TREM2 signal activator.

### Advantageous Effects of Invention

According to the present disclosure, human macrophages that can be induced from hematopoietic progenitor cells in a medium without adding cytokines to the medium, have proliferative capacity after induction, and can be expanded in culture are obtainable.

### Brief Description of Drawings

FIG. 1 is a diagram showing images of macrophage-like adherent cells cultured in the presence of various types of lipids in Example 1;
FIG. 2 is a diagram showing images of bone marrow derived macrophages (BMDMs) cultured in the presence of a macrophage colony-stimulating factor (M-CSF) or a granulocyte-macrophage colony-stimulating factor (GM-CSF) in Comparative Example 1;
FIG. 3 is a diagram showing results of dot plot analysis of BMDMs induced with M-CSF or GM-CSF and human macrophages induced with brain lipid, mycolic acid, or cardiolipin in Test Example 1;
FIG. 4 is a diagram showing results of dot plot analysis of human macrophages induced with L-3-phosphatidylcholine plasmalogen, sulfatide, palmitic acid, stearic acid, or cholesterol in Test Example 1;
FIG. 5 is a diagram showing the number of human macrophages on Day 14 and Day 28 after the start of induction in Test Example 1;
FIG. 6 is a diagram showing changes in the number of macrophages up to Day 28 after the start of induction in Test Example 1;
FIG. 7 is a diagram showing results of evaluation of phagocytic capacity in Test Example 2;
FIG. 8A is a diagram showing the concentration of TNF-α in the supernatant in Test Example 3;
FIG. 8B is a diagram showing the concentration of IL-10 in the supernatant in Test Example 3;
FIG. 9A is a diagram showing the number of cells from Day 9 to Day 57 of culture in Test Example 4; and
FIG. 9B is a diagram showing the fold increase in cell number in Test Example 4.

### Description of Embodiments

Embodiments according to the present disclosure are described below with reference to the drawings. In this regard, the present disclosure is not limited by the below-described embodiments or the drawings. Note that, in the below-described embodiments, the expression, "have", "include", or "contain" encompasses the meaning of "composed of" or "be constituted by".

The method of producing human macrophages according to the present embodiment includes a culture step of culturing hematopoietic progenitor cells in a medium that substantially does not contain either M-CSF or GM-CSF, in the presence of a TREM2 signal activator. TREM2 is an immunoglobulin superfamily receptor that associates with an adaptor molecule, DNAX-activating protein of 12 kDa (DAP12). A TREM2 signal activator may be any substance as long as it specifically binds to TREM2 to generate a signal via DAP12.

The TREM2 signal activator is, for example, an antibody or an antigen-binding fragment thereof, a compound, a ligand of TREM2 or the like, having TREM2 agonist activity or DAP12 agonist activity. The antibody may be either a polyclonal antibody or a monoclonal antibody. Further, the antibody may be a human-type chimeric antibody, a humanized antibody, or a human antibody. The antibody herein includes antigen-binding fragments of an antibody. The antigen-binding fragment is a protein or a peptide containing a portion (partial fragment) of an antibody, and retaining the ability of the antibody to act on and bind to an antigen. Examples of the antigen-binding fragment include F(ab')₂, Fab', Fab, Fab₃, single-chain Fv (scFv), (tandem) bispecific single-chain Fv (sc(Fv)₂), single-chain triplebody, nanobody, divalent VHH, pentavalent VHH, minibody, (double-chain) diabody, tandem diabody, bispecific tribody, bispecific bibody, dual affinity retargeting molecule (DART), triabody (or tribody), tetrabody (or [sc(Fv)₂]₂), and disulfide-bonded Fv (dsFv), and their polymers (see Nature Biotechnology, 29(1): 5-6 (2011); and Christoph Stein and others, Antibodies (1): 88-123 (2012)).

The TREM2 signal activator is, for example, a ligand of TREM2 or a lipid that serves as a TREM2 ligand. Examples of the lipid include sphingoglycolipids, phospholipids, sulfated glycolipids, and fatty acids. For example, the phospholipid may be a lipid that constitutes a biological membrane such as a cell membrane or a mitochondrial membrane. The fatty acid may be either a saturated fatty acid or an unsaturated fatty acid. Preferably, the lipid that serves as a TREM2 ligand is a lipid having multiple, more preferably two to six, or two to four long-chain fatty acid groups. Examples of the long-chain fatty acid group include fatty acid groups having 6 to 60, or 10 to 50 carbon atoms. The number of the carbon atoms constituting the carbon chains of the long-chain fatty acid groups may be either the same or different.

Preferably, examples of the lipid that serves as a TREM2 ligand include mycolic acid (hereinafter also referred to as "MA" simply), cardiolipin (hereinafter also referred to as "CL" simply), L-3-phosphatidylcholine plasmalogen (hereinafter also referred to as "PC" simply), sulfatide (hereinafter also referred to simply as "Sulf' simply), palmitic acid (hereinafter also referred to simply as "Palm"), stearic acid (hereinafter also referred to as "Stea" simply), and cholesterol (hereinafter also referred to as "Chol" simply). These lipids may be naturally derived or synthetically produced. The TREM2 signal activator may be a composition containing a plurality of lipids, such as a composition containing lipids extracted using an organic solvent from animal-derived tissue such as brain, plant-derived tissue, or microorganisms such as bacteria, or a composition in which any two or more kinds of lipids are artificially mixed. Alternatively, the TREM2 signal activator may be a synthetic lipid that binds to TREM2. One aspect of the TREM2 signal activator is a cell containing a lipid on the cell surface, a microorganism, or a microorganism-derived lipid. The TREM2 signal activator is preferably a lipid derived from brain, MA, or CL represented by the following formula as an example. CL is a phospholipid specific to mitochondria.

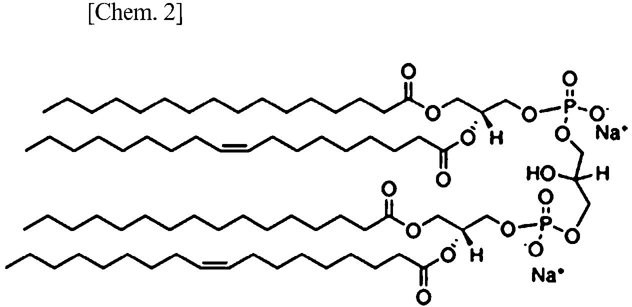

The TREM2 signal activator may be a compound represented by Formula (I), an ester thereof, or a salt thereof. The number of carbon atoms in one molecule of the compound is, for example, 60 to 90, or 70 to 80. R¹ represents a saturated or unsaturated aliphatic hydrocarbon group. The number of carbon atoms of R¹ is, for example, 6 to 60, 10 to 50, 20 to 40, 22 to 30, or 22 to 26. R² represents a saturated or unsaturated aliphatic hydrocarbon group that may contain a ring structure or a substituent. The number of carbon atoms of R² is, for example, 6 to 60, 10 to 50, 20 to 40, 22 to 30, or 22 to 26. The number of carbon atoms constituting the ring structure is, for example, 3 to 6. The ring structure may be contained as a cycloalkylene group in R², or as a cycloalkyl group in R². R² may contain one to several ring structures. Examples of the substituent include, but are not limited to, methyl, hydroxy, carbonyl, and carboxyl.

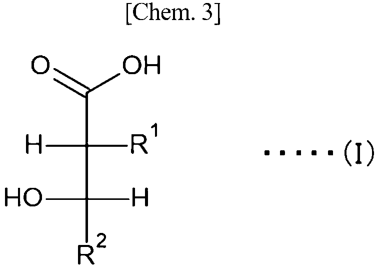

The salt of the compound represented by Formula (I) is not limited as long as it is a pharmaceutically acceptable salt, and may be either an acidic salt or a basic salt. Examples of the salt include alkali metal salts such as lithium salt, sodium salt, and potassium salt; alkaline earth metal salts such as magnesium salt and calcium salt; inorganic acid salts such as hydrochloride, hydrobromide, hydroiodide, sulfate, nitrate, and phosphate; and organic acid salts such as formate, acetate, oxalate, propionate, hexanoate, cyclopentanepropionate, glycolate, pyruvate, lactate, malonate, succinate, malate, fumarate, tartrate, dibenzoyltartrate, ditoluoyltartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, aspartate, camphorsulfonate, glucoheptanoate, 3-phenylpropionate, trimethylacetate, tert-butyl acetate, lauryl sulfate, gluconate, glutamate, hydroxynaphthoate, salicylate, stearate, trifluoroacetate (TFA), maleate, dimaleate, and muconate.

The ester of the compound represented by Formula (I) is not limited as long as it is a pharmaceutically acceptable ester, and examples of the ester include carbonate esters, phosphate esters, nitrate esters, sulfate esters, borate esters, and sulfonate esters. The present embodiment includes various hydrates, solvates, and crystalline polymorphs of the compound of Formula (I) and salts thereof.

The TREM2 signal activator may be selected by applying a substance subject to screening to cells expressing TREM2 and DAP12, and using, as an indicator, activation of the TREM2 signal upon binding of the substance to TREM2, for example, the expression of a reporter protein driven by the TREM2 signal. The cell can be made to express TREM2 and DAP12 by a known method such as introduction of a gene expression plasmid into the cell. Any reporter protein can be used, and examples of the reporter protein include green fluorescent protein (GFP) and the luminescent protein luciferase. Signaling from TREM2 is known to cause expression of genes regulated by transcription factors of the "nuclear factor of activated T cells (NFAT)" type. Therefore, as the cell to be made to express TREM2 and DAP12, a cell containing an NFAT-type transcription factor may be preferably used. Specifically, evaluation of TREM2 signal-activating activity can be carried out using TREM2- and DAP12-expressing cells in which a gene encoding a reporter protein has been introduced as a gene whose expression is regulated by a NFAT-type transcription factor. Whether any lipid serves as a TREM2 ligand can also be confirmed by a similar method.

The term, a "medium that substantially does not contain either M-CSF or GM-CSF" refers to a medium in which at least one of M-CSF and GM-CSF may be present in trace amounts, but neither M-CSF nor GM-CSF is intentionally added. In other words, the term, a "medium that substantially does not contain either M-CSF or GM-CSF" means a medium that contains neither M-CSF nor GM-CSF, or a medium that contains at least one of M-CSF and GM-CSF in an amount insufficient to induce differentiation of human hematopoietic progenitor cells. Examples of a case in which at least one of M-CSF and GM-CSF is present in trace amounts in the medium include cases where the human hematopoietic progenitor cells to be cultured secrete at least one of M-CSF and GM-CSF, or where at least one of M-CSF and GM-CSF is contained in the additives described below.

Examples of the medium include a known medium usable for culturing human hematopoietic progenitor cells, such as an RPMI medium. If necessary, fetal bovine serum (FBS) or the like may be added to the medium. The method of producing human macrophages according to the present embodiment can induce differentiation from human hematopoietic progenitor cells into human macrophages without using cytokines such as M-CSF, GM-CSF, stem cell factor (SCF), thrombopoietin (TPO), Flt3 ligand (FL), IL-6, IL-34, and granulocyte G-CSF. Therefore, preferably, a "medium that substantially does not contain M-CSF or GM-CSF" also substantially does not contain IL-34 or cytokines. The term "substantially does not contain IL-34 or cytokines" means, similarly to the above-described "medium that substantially does not contain either M-CSF or GM-CSF", that IL-34 or cytokines may be present in trace amounts, but are not intentionally added to the medium.

Preferably, a "medium that substantially does not contain M-CSF or GM-CSF" also substantially does not contain one or more kinds of agonists (antibodies, compounds, or the like) that activate cytokine receptors. For example, a "medium that substantially does not contain M-CSF or GM-CSF" substantially does not contain one or more kinds of agonists selected from agonists that activate an M-CSF receptor, agonists that activate a GM-CSF receptor, and agonists that activate an IL-34 receptor. More preferably, a "medium that substantially does not contain M-CSF or GM-CSF" substantially does not contain any of the agonists that activate the M-CSF receptor, the agonists that activate the GM-CSF receptor, and the agonists that activate the IL-34 receptor, and may substantially be free of agonists that activate any cytokine receptors.

Human hematopoietic progenitor cells are cells contained in bone marrow or cord blood in human. The human hematopoietic progenitor cells are, for example, human myeloid progenitor cells. Bone marrow cells collected from human bone marrow, or mononuclear cells collected from umbilical cord blood may be used as the human hematopoietic progenitor cells.

In the culture step, the concentration of the human hematopoietic progenitor cells is not limited. For example, the cells are plated at 1×10³ to 1×10⁷ cells or 1×10⁴ to 1×10⁶ cells per unit culture area (cm²). Preferably, 1×10⁵ human hematopoietic progenitor cells are plated per unit culture area (cm²). The method of producing human macrophages according to the present embodiment can induce differentiation from human hematopoietic progenitor cells into human macrophages without using sustentacular cells or extracellular matrices. Therefore, preferably, in the culture step, the human hematopoietic progenitor cells are cultured under a condition in which neither sustentacular cells nor extracellular matrices are added.

In the culture step, the human hematopoietic progenitor cells can be cultured in a cell culture vessel that retains the TREM2 signal activator. Examples of the cell culture vessel include cell culture plates, cell culture flasks, and cell culture dishes. In cases where the TREM2 signal activator is insoluble in water, and hence insoluble in a liquid medium, the human hematopoietic progenitor cells may be cultured in a cell culture vessel whose surface is coated with the TREM2 signal activator. In cases where a lipid derived from brain is used as the TREM2 signal activator, the mass of the lipid with which the surface of the cell culture vessel is to be coated is, for example, not less than 1.0 µg/cm², 1.5 to 20 µg/cm², 2 to 18 µg/cm², or 3 to 16 µg/cm², preferably 3 to 12 µg/cm², with respect to 1×10⁴ to 1×10⁶ human hematopoietic progenitor cells. The coating of the surface of the cell culture vessel with the TREM2 signal activator may be carried out by dissolving (suspending) the ligand in an organic solvent or the like, adding the resulting solution (suspension) to the cell culture vessel, and then evaporating the organic solvent.

In the culture step, the cells may be cultured by a cell culture method using a known medium. The culture is carried out, for example, under conditions at 37°C at a CO₂ concentration of 5%. During the culture, the medium is preferably replaced, for example, every 3 to 4 days. About 7 days to 10 days after the start of culture, macrophage-like adherent cells are obtained.

The human macrophages obtained by the production method according to the present embodiment can be induced from hematopoietic progenitor cells in a medium without adding cytokines to the medium. Such macrophages are referred to as lipid induced macrophages (LIM). The human macrophages have proliferative capacity after induction and can be expanded in culture. The number of days the human macrophages can be cultured (survival period) is at least 28 days.

Another embodiment provides human macrophages (lipid induced macrophages) whose differentiation is induced dependently on TREM2 by culturing human hematopoietic progenitor cells in a medium that substantially does not contain either M-CSF or GM-CSF, preferably under a condition in which neither sustentacular cells nor extracellular matrices are added, in the presence of a TREM2 signal activator.

As shown in Test Example 1 below, the human macrophages according to the present embodiment had proliferative capacity at least after Day 28 of successive culture under a condition in which the medium did not contain either M-CSF or GM-CSF, and neither sustentacular cells nor extracellular matrices were added, in the presence of a TREM2 signal activator. Therefore, another embodiment provides human macrophages having proliferative capacity after the start of induction, on Day 14 or more, preferably Day 20 or more, or Day 28 or more of successive culture under a condition in which the medium does not contain either M-CSF or GM-CSF, and contains a TREM2 signal activator, preferably without adding both sustentacular cells and extracellular matrices.

The human macrophage according to the present embodiment may have any one, any two or more, any three or more, any four or more, any five or more, or all characteristics selected from the characteristics described above.

Another embodiment provides a method of preparing human macrophages, a method of manufacturing human macrophages, a method of inducing differentiation into human macrophages, a method of culturing human macrophages, or a method of proliferating human macrophages, including the culture step described above. The method of culturing human macrophages or the like enables maintenance of the human macrophages for a long period. The method of proliferating human macrophages allows proliferation of the human macrophages. Human macrophages can be expanded in culture in a medium that substantially does not contain either M-CSF or GM-CSF in the presence of a TREM2 signal activator. Accordingly, another embodiment provides a method of proliferating human macrophages, the method including a culture step for culturing human macrophages in a medium that substantially does not contain either M-CSF or GM-CSF in the presence of a TREM2 signal activator.

Another embodiment provides a differentiation-inducing agent that contains the TREM2 signal activator, but substantially does not contain either M-CSF or GM-CSF. The differentiation-inducing agent induces differentiation from human hematopoietic progenitor cells into human macrophages. The differentiation-inducing agent may include a solvent, water, ethanol, a polyol, an oil component, a surfactant, a thickener, an antiseptic, a pH-adjusting agent, or the like in addition to the TREM2 signal activator. The differentiation-inducing agent may be in the form of, for example, a liquid, a gel, a cream, or a solid. Preferably, the differentiation-inducing agent is used in a state where the agent is added to a cell culture vessel, or where the agent is applied to the surface of a cell culture vessel to coat the surface of the vessel. The differentiation-inducing agent enables production of human macrophages that have a long survival period from hematopoietic progenitor cells.

Another embodiment provides a proliferation-promoting agent for promoting proliferation of human macrophages, where the proliferation-promoting agent contains the TREM2 signal activator, but substantially does not contain either M-CSF or GM-CSF. The proliferation-promoting agent promotes proliferation of human macrophages, in particular, human macrophages obtained by the production method described above. The proliferation-promoting agent may include components other than the TREM2 signal activator, similarly to the differentiation-inducing agent.

Another embodiment provides a differentiation-inducing kit including a cell culture vessel having a surface coated with a TREM2 signal activator. The differentiation-inducing kit is used for culturing human hematopoietic progenitor cells in a medium that substantially does not contain either M-CSF or GM-CSF, and induces differentiation from human hematopoietic progenitor cells into human macrophages. The differentiation-inducing kit may also include a medium such as a basal medium, a low-serum medium, or a serum-free medium; a serum required for cell culture, such as FBS; or another known additive to be added to a medium.

Since the differentiation-inducing kit includes a cell culture vessel having a surface preliminarily coated with a TREM2 signal activator, human macrophages that have a long survival period can be simply obtained merely by plating human hematopoietic progenitor cells in the cell culture vessel and culturing those cells. The differentiation-inducing kit can be used also as a proliferation-promoting kit for promoting proliferation of human macrophages.

The present disclosure is more specifically described with reference to the following Examples, but the present disclosure is not limited to the Examples. Examples

### Example 1: Preparation of Human Lipid-Induced Macrophages (LIMs)

[Coating of Cell Culture Plate with Lipid] A test substance, mouse brain lipid (hereinafter also referred to as "BL" simply), MA, CL, PC, Sulf, Palm, Stea, or Chol, was diluted with methanol to 50 µg/ml. The test substance was dispensed at 100 µl (5 µg) portions into a 24-well cell culture plate, and then the plate was left to stand in a clean bench, to evaporate the methanol. It is known that PC and Sulf are ligands of human TREM2 (Yaming Wang and 12 others, "TREM2 Lipid Sensing Sustains the Microglial Response in an Alzheimer's Disease Model", Cell, 2015, 160, 1061-1071).

The BL was prepared as described below. Mouse brain (about 400 mg/mouse) was immersed in 1 ml of methanol in a polypropylene tube, and minced with scissors. The entire amount of the obtained sample was transferred to a glass test tube, and 1 ml of methanol and 4 ml of chloroform were added thereto to achieve a chloroform-to-methanol (MeOH) volume ratio of 2:1 (C-M 2:1). The sample was mixed for 3 hours at room temperature using a shaker (200 rpm). The sample was centrifuged at 2000 rpm for 15 minutes, and then the supernatant was filtered and collected into a glass bottle, to obtain Extract 1. To the residual brain in the glass test tube, 2 ml of methanol and 4 ml of chloroform were newly added, and the resulting sample was mixed for 16 hours at room temperature using a shaker (200 rpm). After filtration, the supernatant was collected into the glass bottle containing Extract 1, and the resulting mixture was mixed well, to obtain Extract 2. Extract 2 was stored at -30°C until use. Into a weighed glass bottle, 1.8 ml of Extract 2 was dispensed, and the solvent was evaporated by blowing nitrogen under heat at 37°C, followed by measuring the dry weight of lipid. The lipid was dissolved in C-M 2:1 at a concentration of 5 mg/ml, to prepare a BL extract.

[Induction of LIM] Collected human bone marrow cells were suspended in 10% FBS/RPMI at 4×10⁵ cells/ml. The bone marrow cell suspension was added to the cell culture plate coated with the test substance, at 1×10⁵ cells/0.25 ml/cm². The bone marrow cells were cultured at 37°C in a 5% CO₂ incubator. On Day 3 of the culture, fresh medium in a half amount was added to the culture plate. Thereafter, the medium was replaced every 3 to 4 days.

### Results:

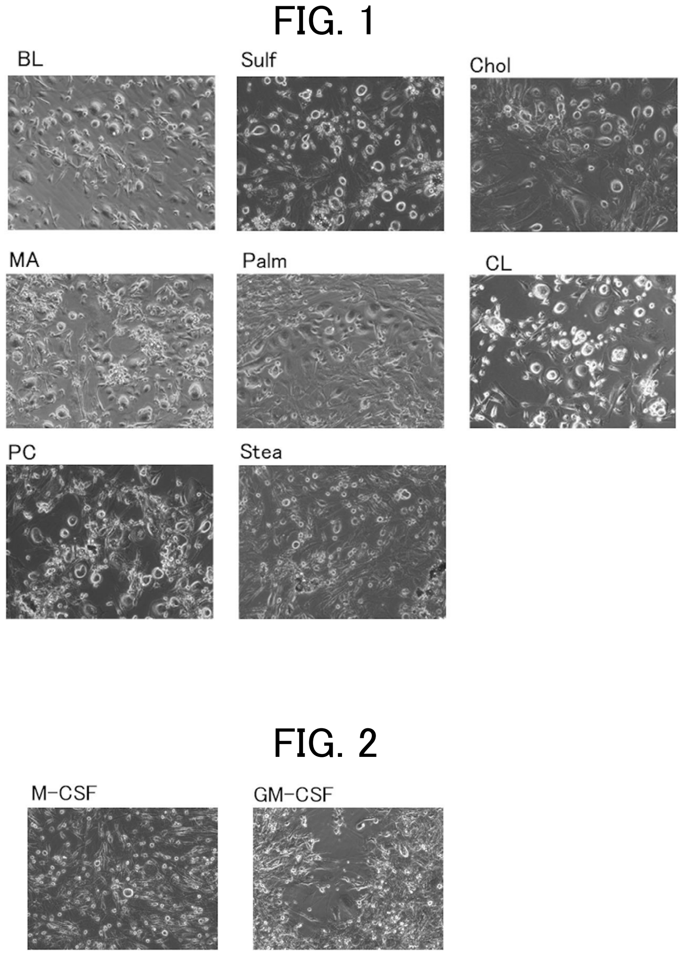

About 7 days to 10 days after the start of culture, macrophage-like adherent cells have begun to appear. FIG. 1 shows images of LIMs on Day 14 after the start of culture.

### Comparative Example 1: Preparation of BMDMs

Collected human bone marrow cells were suspended in 10% FBS/RPMI at 4×10⁵ cells/ml. To the resulting cell suspension, 100 ng/ml M-CSF or GM-CSF was added. To each well of a 24-well culture plate, the bone marrow cell suspension was added at 2×10⁵ cells/0.5 ml. The bone marrow cells were cultured at 37°C in a 5% CO₂ incubator. On Day 3 of the culture, 100 ng/ml M-CSF or GM-CSF/10% FBS/RPMI in a half amount (0.25 ml/well) was added. On Day 5 of the culture, the supernatant was aspirated, and 0.5 ml/well of 10% FBS/RPMI was added, followed by detaching the cells using a scraper. After 4-fold dilution with 10% FBS/RPMI, the resulting dilution was dispensed at 0.5 ml/well, and 100 ng/ml M-CSF or GM-CSF was added thereto. Thereafter, half the medium was replaced with 100 ng/ml M-CSF/10% FBS/RPMI every 3 to 4 days.

### Results:

On Day 14 after the start of culture, the cells increased to 2.1 to 8.7×10⁴ cells/well. FIG. 2 shows images of BMDMs on Day 14 after the start of culture.

### Test Example 1: Expression Analysis of Cell Surface Markers of BMDMs and LIMs

LIMs on Day 14 and Day 28 after the start of induction in Example 1 and BMDMs on Day 14 and Day 28 after the start of induction in Comparative Example 1 were separately collected by detaching the cells together with the medium using a cell scraper, and the cells were counted using a hemocytometer. Centrifugation was carried out at 500 G at 4°C for 5 minutes, and then the supernatant was discarded. 1% FBS/HBSS was added to the cells to prepare a suspension at 1×10⁷ cells/ml. Human TruStain FcX (manufactured by BioLegend) was added to the cell suspension at 1/20 volume, and the resulting mixture was left to stand on ice for 10 minutes, to perform blocking of Fcy receptors.

The cell suspension after the blocking was dispensed into a 96-well V-bottom plate at 10 µl/well. PE anti-mouse/human TREM2 antibody (manufactured by R&D Systems), FITC anti-mouse/human CD11b antibody, APC anti-human CD163 antibody, APC/Cyanine7 anti-human CD45 antibody, and PE/Cyanine7 anti-human CD115 antibody (all of these were manufactured by BioLegend) diluted with 1%FBS/HBSS was added to the cell suspension such that the recommended concentration described in the manufacturer's instructions was achieved in a total volume of 20 µl/well, followed by leaving the resulting mixture to stand on ice for 15 minutes to allow the reaction to proceed. To wash the sample, 180 µl/well of 1% FBS/HBSS was added.

The sample was centrifuged at 500 G at 4°C for 5 minutes, and then the supernatant was discarded. To wash the sample, 180 µl/well of 1% FBS/HBSS was added. The sample was centrifuged at 500 G at 4°C for 5 minutes, and then the supernatant was discarded. The sample was suspended by adding 50 µl/well of 1% FBS/HBSS. The obtained sample was analyzed by flow cytometry. CD45-positive CD11b-positive CD115-positive CD163-positive cells were extracted as macrophage cells, subjected to analysis, and counted.

### Results:

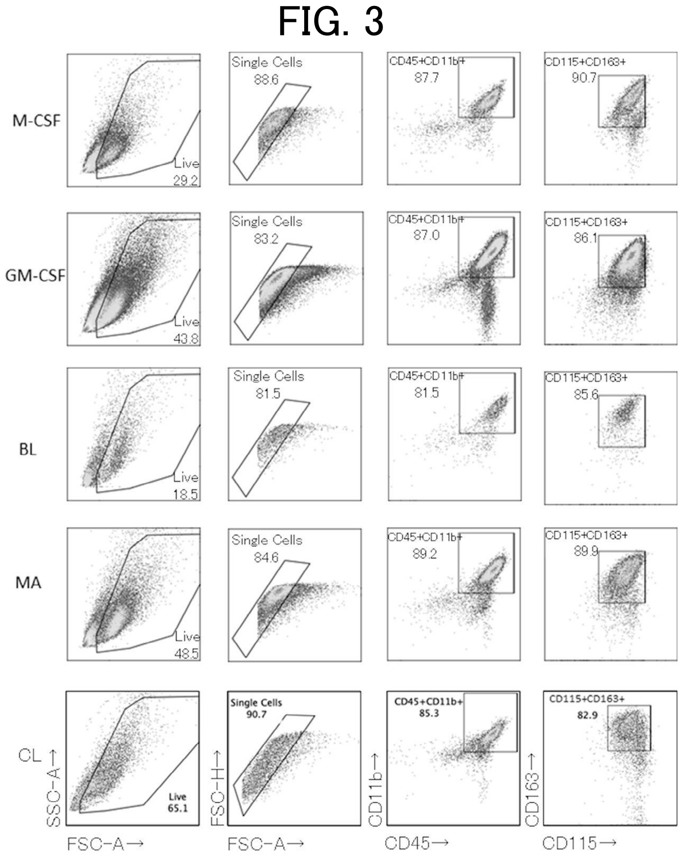

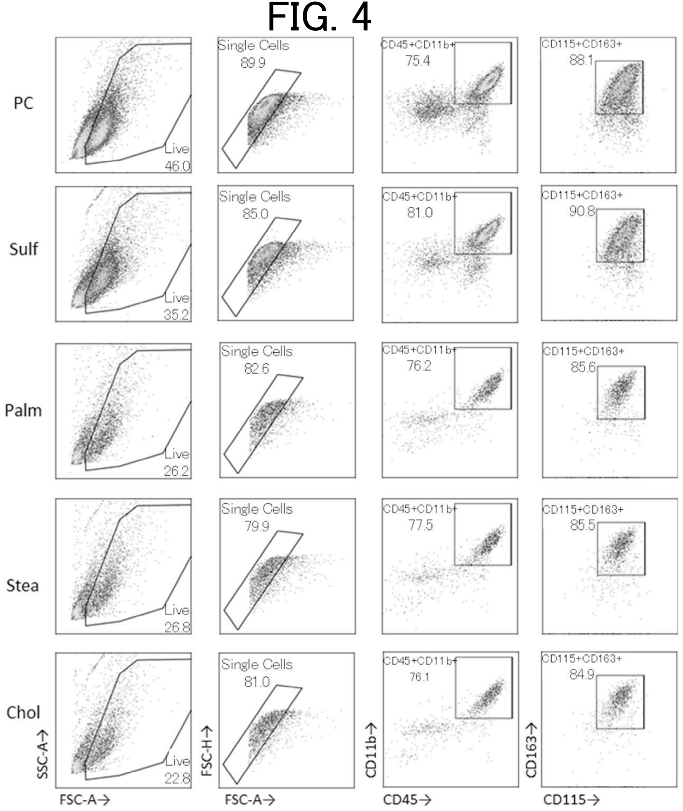

FIG. 3 shows the results of dot plot analysis of BMDMs induced with M-CSF or GM-CSF, and LIMs induced with BL, MA, or CL. FIG. 4 shows the results of dot plot analysis of LIMs induced with PC, Sulf, Palm, Stea, or Chol. The LIMs and the BMDMs had similar signal distributions regarding the forward scatter (FSC), which is an index of the cell size, and the side scatter (SSC), which is an index of the complexity of cells (number of granules). The LIMs showed high expression of CD45, a pan-leukocyte marker, and CD11b, a marker for granulocytes and macrophages, as well as high expression of CD115, a monocyte marker, and CD163, a macrophage marker. From the results above, the LIMs were shown to be macrophages.

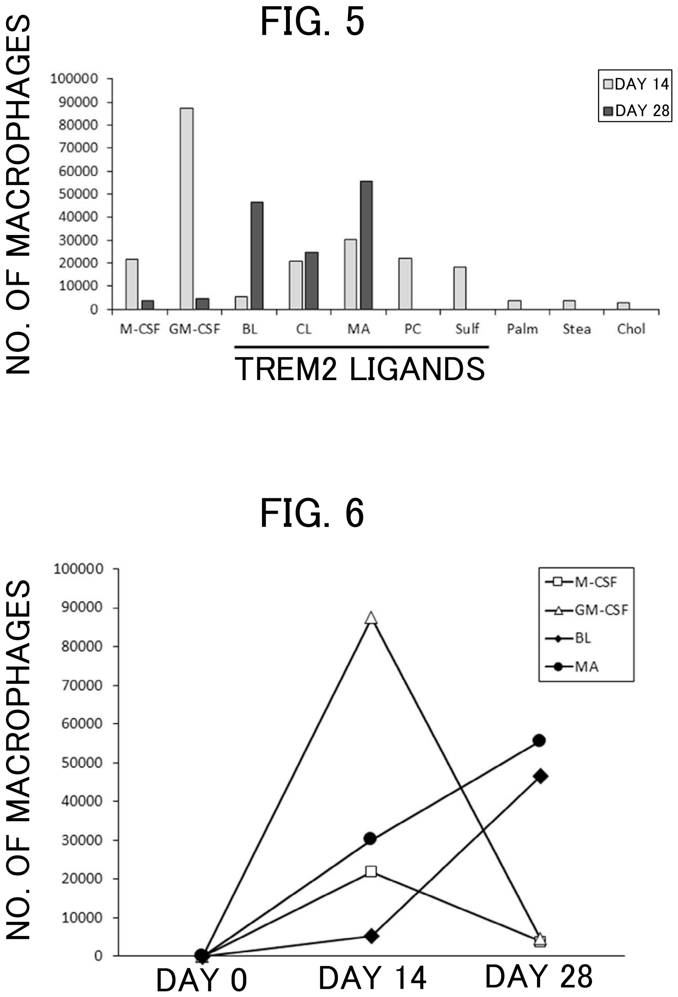

FIG. 5 shows the number of macrophages on Day 14 and Day 28 after the start of induction. In addition to CL, PC, and Sulf that have been reported as ligands of TREM2, BL and MA were shown to induce LIMs.

FIG. 6 shows changes in the number of macrophages up to Day 28 after the start of induction. The number of BMDMs induced with M-CSF or GM-CSF decreased compared to Day 14, whereas the number of LIMs induced with BL or MA continued to increase.

### Test Example 2: Evaluation of Phagocytic Capacity

BMDMs induced with M-CSF or LIMs induced with BL were plated in a 96-well plate at 1×10⁴ cells/100 µl/well, and 5 µg of pHrodo Green E. coli BioParticles (manufactured by Thermo Fisher Scientific) was added thereto, followed by incubation at 37°C for 3 hours. After the incubation, the cells were collected and fluorescence intensity of pHrodo Green was measured by flow cytometry, to evaluate the phagocytic capacity.

### Results:

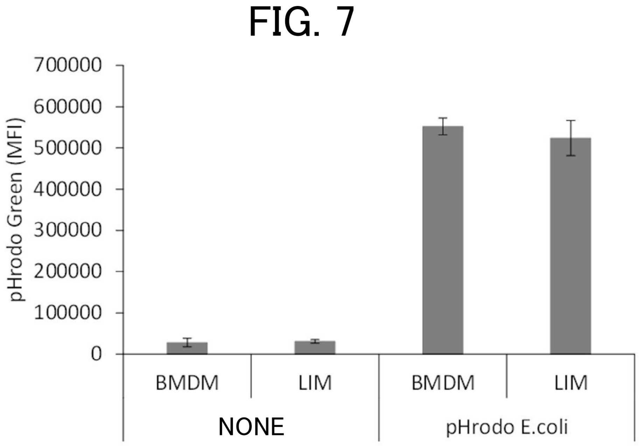

FIG. 7 illustrates the mean fluorescence intensity (MFI) associated with the phagocytosed pHrodo Green E. coli BioParticles. LIMs exhibited phagocytic capacity comparable to that of the BMDMs.

### Test Example 3: Study of Cytokine Production Response to Lipopolysaccharide (LPS) Stimulation

BMDMs induced with M-CSF or LIMs induced with BL were plated in a 96-well plate at 2×10⁴ cells/100 µl/well, and 10 ng/ml LPS was added thereto, followed by incubation at 37°C for 24 hours. The culture supernatant was collected after the incubation, and the concentrations of TNF-α and IL-10 in the supernatant were measured using ELISA kits (ELISA MAX Standard Set Human TNF-α and ELISA MAX Standard Set Human IL-10, manufactured by BioLegend).

### Results:

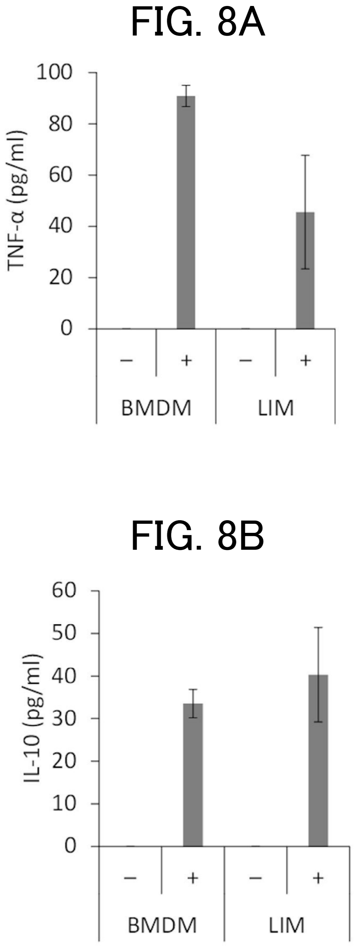

FIGS. 8A and 8B respectively show the concentration of TNF-α and the concentration of IL-10 in the supernatant. LIMs exhibited cytokine production activity comparable to that of the BMDMs.

### Test Example 4: Successive Culture of LIMs

In cases where a 24-well plate is used, macrophages appeared around Day 9 after the start of induction. Therefore, BMDMs induced with M-CSF or GM-CSF or LIMs induced with BL or CL were serially cultured every 14 to 18 days as follows. After aspiration of the culture supernatant, 0.1 ml of 1 mM ethylenediaminetetraacetic acid (EDTA)/phosphate-buffered saline (PBS) was added, followed by incubation at 37°C for 5 minutes. Then, 0.4 ml of 10% FBS/RPMI was added, and the cells were detached by pipetting and using a cell scraper and suspended. The number of cells was counted using a hemocytometer. The cell suspension was diluted 4-fold with 10% FBS/RPMI, and 0.5 ml/well of the resulting dilution was dispensed into a new lipid-coated plate. Thereafter, half of the 10% FBS/RPMI medium was replaced every 3 to 4 days, and the cells were serially cultured again on a day from Days 14 to 18.

### Results:

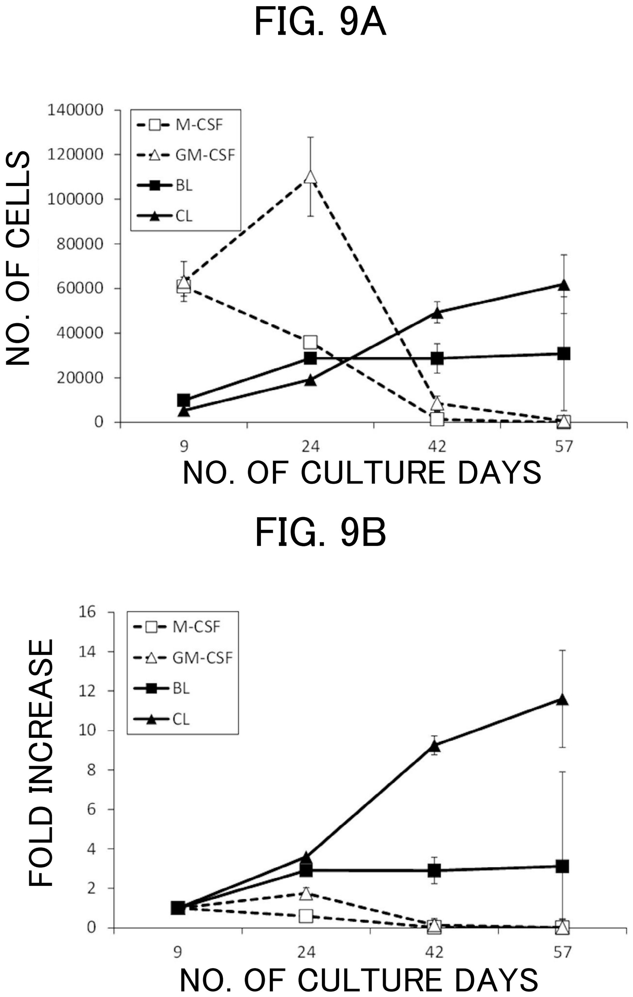

FIG. 9A is a diagram showing the number of cells from Day 9 to Day 57 of culture. FIG. 9B shows the fold increases in the number of cells during Day 9 to Day 57 of the culture relative to the number of cells of Day 9 that is taken as 1. The BMDMs induced with M-CSF or GM-CSF hardly increased after the successive dilution. In contrast, the LIMs induced with BL or CL survived and proliferated for more than 57 days in the culture.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-101497, filed on June 21, 2023, the entire disclosure of which is incorporated by reference herein.

### Industrial Applicability

The present disclosure is useful for obtaining human macrophage.

## Claims

1. A method of producing human macrophages, the method comprising:
culturing human hematopoietic progenitor cells in a medium that substantially does not contain either a macrophage colony-stimulating factor or a granulocyte-macrophage colony-stimulating factor, in presence of a TREM2 signal activator.

2. The method according to claim 1, wherein
the medium substantially does not contain interleukin-34.

3. The method according to claim 1 or 2, wherein
the medium substantially does not contain cytokine.

4. The method according to claim 1 or 2, wherein
in the culturing, the human hematopoietic progenitor cells are cultured under a condition in which neither a sustentacular cell nor an extracellular matrix is added.

5. The method according to claim 1 or 2, wherein
the TREM2 signal activator is a lipid.

6. The method according to claim 1 or 2, wherein
the TREM2 signal activator is a lipid derived from brain or a lipid having a plurality of long-chain fatty acid groups.

7. The method according to claim 1 or 2, wherein
the TREM2 signal activator is a compound represented by Formula (I), an ester thereof, or a salt thereof (wherein the compound has 60 to 90 carbon atoms, R¹ represents a saturated or unsaturated aliphatic hydrocarbon group, and R² represents a saturated or unsaturated aliphatic hydrocarbon group optionally containing a ring structure or a substituent)

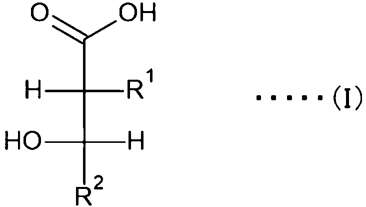

8. The method according to claim 1 or 2, wherein
the TREM2 signal activator is a phospholipid.

9. The method according to claim 8, wherein
the phospholipid is a lipid that constitutes a biological membrane.

10. The method according to claim 8, wherein
the phospholipid is cardiolipin.

11. The method according to claim 1 or 2, wherein
a surface of a cell culture vessel for culturing the human hematopoietic progenitor cells is coated with the TREM2 signal activator.

12. The method according to claim 1 or 2, wherein
the human hematopoietic progenitor cells are human myeloid progenitor cells.

13. A differentiation-inducing agent that induces differentiation from human hematopoietic progenitor cells into human macrophages, the differentiation-inducing agent comprising:
a TREM2 signal activator, wherein the differentiation-inducing agent substantially does not contain either a macrophage colony-stimulating factor or a granulocyte-macrophage colony-stimulating factor.

14. A differentiation-inducing kit that induces differentiation from human hematopoietic progenitor cells into human macrophages, the differentiation-inducing kit comprising:
a cell culture vessel having a surface coated with a TREM2 signal activator, wherein
the differentiation-inducing kit is used for culturing the human hematopoietic progenitor cells in a medium that substantially does not contain either a macrophage colony-stimulating factor or a granulocyte-macrophage colony-stimulating factor.

15. A method of inducing differentiation into human macrophages, the method comprising:
culturing human hematopoietic progenitor cells in a medium that substantially does not contain either a macrophage colony-stimulating factor or a granulocyte-macrophage colony-stimulating factor, in presence of a TREM2 signal activator.

16. A proliferation-promoting agent that promotes proliferation of human macrophages, the proliferation-promoting agent comprising:
a TREM2 signal activator, wherein the proliferation-promoting agent substantially does not contain either a macrophage colony-stimulating factor or a granulocyte-macrophage colony-stimulating factor.

17. A proliferation-promoting kit that promotes proliferation of human macrophages, the proliferation-promoting kit comprising:
a cell culture vessel having a surface coated with a TREM2 signal activator, wherein
the proliferation-promoting kit is used for culturing human hematopoietic progenitor cells in a medium that substantially does not contain either a macrophage colony-stimulating factor or a granulocyte-macrophage colony-stimulating factor.

18. A method of proliferating human macrophages, the method comprising:
culturing human hematopoietic progenitor cells in a medium that substantially does not contain either a macrophage colony-stimulating factor or a granulocyte-macrophage colony-stimulating factor, in presence of a TREM2 signal activator.

19. A human macrophage, capable of proliferating after 10 days or more of successive culture in a medium that substantially does not contain either a macrophage colony-stimulating factor or a granulocyte-macrophage colony-stimulating factor, in presence of a TREM2 signal activator.

20. A human macrophage, of which differentiation is induced dependently on TREM2 by culturing human hematopoietic progenitor cells in a medium that substantially does not contain either a macrophage colony-stimulating factor or a granulocyte-macrophage colony-stimulating factor, in presence of a TREM2 signal activator.
